Europäisches Patentamt

European Patent Office

Office européen des brevets

(11) Publication number: **0 128 018 B1**

# EUROPEAN PATENT SPECIFICATION

(45) Date of publication of patent specification: **15.07.92**    (51) Int. Cl.⁵: **C12Q 1/68**

(21) Application number: **84303671.6**

(22) Date of filing: **31.05.84**

(54) **Molecular genetic probe, and method of forming same, assay technique and kit using said molecular genetic probe.**

(30) Priority: **31.05.83 US 499440**

(43) Date of publication of application:
**12.12.84 Bulletin 84/50**

(45) Publication of the grant of the patent:
**15.07.92 Bulletin 92/29**

(84) Designated Contracting States:
**AT BE CH DE FR GB IT LI LU NL SE**

(56) References cited:
**EP-A- 063 879**
**WO-A-83/02286**
**GB-A- 2 019 408**
**US-A- 4 358 535**

**BIOCHEMISTRY, vol. 17, no. 26, 1978, pages
5791-5798, American Chemical Society, US;
W.E. STUMPH et al.: "Gene enrichment using
antibodies to DNA/RNA hybrids: purification
and mapping of Dictyostelium discoideum
rDNA"**

(73) Proprietor: **Orgenics Ltd.**
**P.O. Box 360**
**Yavne 70650(IL)**

(72) Inventor: **Herzberg, Max**

**Moshav Sataria 73272(IL)**

(74) Representative: **Clifford, Frederick Alan et al
MARKS & CLERK 57/60 Lincoln's Inn Fields
London WC2A 3LS(GB)**

Description

The present invention relates to a molecular genetic probe and a method of forming the same, to an assay technique and a kit using the said molecular genetic probe.

The probe may be used as part of a DNA- or RNA-based assay technique. More specifically, the molecular genetic probe of the invention finds particular use in assay techniques for determining the presence of a virus gene, a gene or nucleic acid sequence, or any gene product, e.g., a coding sequence for an enzyme, in a suspect sample containing a suspect nucleic acid wherein the suspect nucleic acid is hybridized to the probe and complexed with a labelling agent whose presence can be quantitatively assayed.

The assay techniques outlined above are of obvious diagnostic value and are of particular value when embodied in kit form such that the said kits can be used and the said techniques performed easily and simply, by lab personnel having only limited laboratory skills. It should however be noted that the techniques of the present invention are not limited to their use in diagnostic methods.

There is a particular demand for such techniques, suitable for kits, which are capable of assaying the presence of a particular pathogen; which are specific, and which operate by sensing the presence of nucleic acid specific to the suspect pathogen. Obviously, it is preferable that such techniques be not only accurate, but that they also require performance of a minimum number of manipulations, both by the laboratory technician, as well as in the manufacture of the reagents used in the assay.

It is desirable that the principle of the assay be based upon detection of DNA, or RNA, since such a technique can be used to detect disease, even when symptons are not apparent. It should be noted that DNA (or in some cases RNA) is a nucleotide polymer which contains the genetic information characteristic of a particular organism. By detecting the presence of specific sequences of the polymer it is possible to determine the presence of the organism to which those sequences are specific. Such techniques are of particular interest in pathogen assays for use in viral diseases wherein one wishes to determine the presence of a particular virus in particular tissue.

In situ techniques exist for assaying for the presence of particular viral DNA and RNA by using hybridization techniques in which probe DNA capable of hybridizing with the suspect viral DNA is radioisotopically tagged and then added to the test specimen. Standard counting and imaging techniques may then be used to quantitatively assay for the presence and amount of suspect DNA which is present.

Such a technique is broadly disclosed in U.S.P. 4,385,535 to Falkow et al. in which denatured suspect DNA (or RNA), is contacted with single stranded DNA (RNA) probes which have been labelled. A special denaturation technique is disclosed. The probe is selected to hybridize with the denatured suspect DNA. The screening technique is said to be suitable for use in screening viruses (genital Herpes is specifically mentioned), fungi, protozoa, molds, etc. The label used is generally suggested to be a radionuclide, however the patent mentions that in certain instances it may be feasible to employ antibodies which bind specifically to the probe for detection purposes. In such instances the antibodies themselves are labelled. Labels listed include radioactive labels, and ligands which can serve as a specific binding member to a labelled antibody, fluorescers, chemiluminescers, enzymes, antibodies which can serve as a specific binding pair member for a labelled ligand, and the like.

European Patent Application No. 0062286 discloses a method and test kit for the detection of Hepatitis B virus by nucleic acid hybridization. Once again a hybridization probe is used which can then be assayed by a scintillation counting technique.

Russian Patent 649,751 discloses a method for identifying microorganisms by purifying DNA from a microorganism and hybridizing this DNA to DNA from a reference strain. The DNA is labelled with radioactive thymine.

One drawback of such systems is the necessity of handling radioactive materials during the preparation of the probes which is obviously unsatisfactory as these materials are both short-lived and potentially hazardous. Another disadvantage which appears in these techniques is the relatively sophisticated equipment which is necessary for such an enzymatic process.

Both United States Patents 4,302,204 (Wahl et al.) and 4,139,346 (Rabbani et al.) also disclose hybridization techniques in which RNA and DNA strands are hybridized with labelled probes as a part of a hybridization technique.

It should be noted that DNA per se is not a satisfactory antigen for purposes of the screening technique which is contemplated. The antibody response of viral DNA is often insufficient and not sufficiently specific to render it usable as an antigen.

The modification of DNA and the use of the modified antigen to elicit an antibody response are known techniques.

An article entitled: "Antibodies Specific for Modified Nucleosides: An Immunochemical Approach for the Isolation and Characterization of Nucleic Acids" by Munns and Liszewski, Progress in Nucleic Acid Research and Molecular Biology, Vol. 24, pp. 109-165 (1980), discusses antibodies specific to modified constituents of nucleic acid.

"Immunological Detection of 06-Methylguanine in Alkylated DNA" by Briscoe et al., Biochemistry, Vol. 17, pp. 1896-1901 (1978) describes the potential use of antibodies specific for 06-methylguanine which bind directly to alkylated DNA. A radioimmunoassay system employing these antibodies is said to be considered to be a useful approach in detecting 06-methylguanine in DNA treated with certain alkylating agents.

"Reactivity of Antibodies to DNA Modified by the Carcinogen N-Acetoxy-N-acetyl-2-Aminofluorene", by Sage et al., Biochemistry, Vol. 18, pp. 1328-1332 (1979) describes the reactivity of antibodies to DNA which were modified by N-Acetoxy-N-acetyl-2-Aminofluorene". These antibodies react with the modified DNA, rather than the unmodified form.

"Possible Relevance of 0-6 Alkylation of Deoxyguanosine to the Mutagenicity and Carcinogenicity of Nitrosamines and Nitrosamides", by Loveless, Nature, Vol. 223, July 12 (1969) discusses the use of biological alkylating agents such as N-ethyl-N-nitrosourea which modify nucleic acid bases.

Thus, although modification, including ethylation of nucleic acid bases is a recognized phenomenom, the advantages of using this principle in conjunction with hybridization, as part of an efficient assay technique, have not generally previously been appreciated or used to maximum advantage. In fact, while hybridization has been suggested in at least one instance, for use in conjunction with modification in one assay procedure, this procedure does not combine the two techniques to maximum advantage and results in a very cumbersome assay having only limited utility.

Such a technique is seen in European Application S.N. 82301804.9 (published as EP-A-0063879 on November 3, 1982) which discloses modifying the purine or pyrimidine bases by convalently bonding a moiety consisting of at least three carbon atoms (e.g. biotin) which is capable of forming a detectable complex with a polypeptide when the compound is incorporated into a double stranded RNA/DNA heteroduplex or DNA-RNA hybrid. The polypeptide detectors for the biotin-containing probe may be avidin, streptavidin or an antibiotin immunoglobulin. In particular, the preferred protein for biotin-like probe detection is the antibiotin immunoglobulin. This antibody can be tagged with an enzyme, such as peroxidase. Page 30, line 37 through page 31, lines 1-14 describe the use of an antibody to detect the biotin constituent. An illustration is provided at page 33 which describes the use of an antibody to detect the biotin or hapten. The IgG-peroxidase example describes an antibody-tagged enzyme detection system. On page 23, lines 25-37 and page 35, lines 1-2, the procedure for utilizing the modified nucleotides to detect the presence of the nucleotide sequences of interest is described. This general protocol can be applied to the detection of nucleic acid sequences of viral, bacterial, fungal or parasite origins in clinical samples.

As was noted above, this technique is of only limited utility and is extremely cumbersome. Using this technique, nuclease treatment of the template nucleic acid is necessary to nick or open gaps in one of the strands. A biotin substituted base is then inserted into the DNA using polymerase. The modified DNA can only then be complexed with antibiotin which is enzyme tagged to form an antibiotin-enzyme-DNA complex. The double stranded DNA can then be split, with the complexed strand being usable as a probe. Such a technique thus requires that modification occur on DNA in its double stranded form only. The modified single strands can then be used to hybridize only with organisms that contain double stranded nucleic acids, i.e., organisms such as the single stranded RNA viruses cannot be modified or detected using the approach proposed in this patent application.

Furthermore, the system requires additional reagents such as nuclease and polymerase and the prior synthesis of the biotin base. The use of these substances not only adds to the complexity of the technique but also requires high purity. Also, the use of enzymes which break and then reform the modified nucleic acid chain add to the time and expense involved in manufacturing the kit reagents, further reducing the value of this technique for use in diagnostic kits.

Pages 62-63 of the patent disclose the modification, by addition, of a polynucleotide. Using a mercuric salt a mercurated derivative is formed. The mercurated derivative is then reacted with a linker arm and a reactive terminal functional group or the modifier itself in the presence of $K_2PdCl_4$. Thus, at least two, if not three, steps are required to perform the modification.

Such a procedure is unsatisfactory because it necessarily requires purification between each step. Also, the use of mercury is a strong disadvantage because it renders the process unusable for double stranded systems due to intercalation (there are known techniques for marking a double stranded polynucleotide by intercalation). Furthermore, as discussed, a linker arm is necessary because of the position on which the moiety is added. A technique which did not require a linker arm would be much more satisfactory. Yet further, using the technique of the publication, only one addition per base is possible, whereas multiple addition per base may sometimes be desirable.

It is, therefore, an object of the present invention to provide an assaying or screening technique which is antibody-specific, which is inexpensive, for which the reagents can be easily synthesized, and which has broad utility in assaying nucleic acids from organisms which are both single and double stranded, with little concern for purity.

It is a further object of the invention to provide a technique in which a strong antigen is used, e.g., whereby a strong antibody response is elicited, but which does not nevertheless substantially interfere with hybridization of the probe to the suspect nucleic acid.

Yet another object of the invention is to provide a technique which obviates the use of radionuclides, if one is desirous of minimizing exposure to such materials, and increasing the shelf life of the reagents.

According to yet another objective of the invention, it would be desirable to provide a technique for forming the probe which involves only a one-step addition of the modifying agent, which does not use mercury and allows for addition of more than one modifying agent into a single base on a nucleic acid.

According to the first aspect of the present invention, there is provided a method assaying for the presence or quantity of a suspect nucleic acid in a suspect sample containing a single stranded nucleic acid sequence, comprising the steps of:

(a) contacting said suspect sample with a modified nucleic acid probe containing a single stranded nucleic acid sequence, complementary to a nucleic acid sequence of the suspect nucleic acid, to form a hybridized complex, said modified nucleic acid probe having been formed by a one-step modification process selected from the group consisting of alkylation, sulfonation, nitrosonation, or nitrophenylation;

(b) contacting said hybrydized complex with antibodies specific to the modified portion of said modified nucleic acid probe to form an antibody-bound complex; and

(c) measuring for the presence and/or extent of the presence of said antibodies as an indication of the presence or extent of said suspect nucleic acid.

Using such a technique, nucleic acid from organisms containing single, a well as double, stranded DNA and RNA may be assayed, under conditions of less than high purity, which might otherwise be required.

Also, using such a technique a label may be selected which is non-radioactive, thus rendering it easily usable under conditions where one wishes to avoid the handling of radioactive substances. The label may be an enzyme directly or, for example, a second antibody to the first antibody which is itself complexed with an enzyme.

It is a particular advantage of this technique that the DNA or RNA probe itself may be made by direct modification, without enzymatic or other types of nucleic acid manipulation techniques. Specifically, the use of nuclease and polymerase may specifically be avoided.

The probes themselves are either single stranded or double strands which have been at least partially denatured, that is, in the case of the double strands the duplex has been partially separated. The primary requirement of the probe is that it be capable of hybridizing with the suspect nucleic acid, when modified.

The particular modification of the DNA or RNA involves modifying nucleic acid bases, and particularly in one preferred embodiment, alkylating the bases. Alkylation may be performed by using an alkylation compound such as alkylnitrosourea, and even more specifically, methyl, ethyl or propyl nitrosourea compounds. In one particularly preferred embodiment the alkylation compound is N-ethyl-nitroso-urea. Quite obviously other alkylation compounds eliciting similar antibody responses in substantially the same manner may also be used without departing beyond the scope of the invention.

Alkylation of the $O_6$ position of guanidine is preferred although other locations may also be alkylated depending upon the particular compound being used, see Briscoe et al., supra. for one example of an addition modification reaction which may be used according to the invention.

According to another embodiment of the invention, modification may take the form of sulfonation. Such a sulfonation technique is disclosed in "Immunochemical Approaches to DNA Structure Investigation - I", by A. M. Poverenny et al., Molecular Immunology, Vol. 16, pp. 313-316, Pergamon Press, Ltd., 1979.

Other types of addition reaction modifications, such as nitrosonation, nitrophenylation, etc, may also be used without departing from the scope of the invention.

The invention technique lends itself readily for use in a kit which tests for the presence of a nucleic acid in a suspect sample. Such a kit includes a supply of modified nucleic acid probes selected such that they will hybridize with the suspect nucleic acid, if it is present, to form a hybridized complex. The modified nucleic acid itself is formed by modification of a base of the probe nucleic acid. Means are provided for contacting the suspect sample with the modified nucleic acid to form the hybridized complex. A supply of antibodies selected to complex with at least the modified portion of the modified nucleic acid is provided. The antibodies are labelled with a labelling agent adapted to signal the presence of the natibody. The kit further includes means for contacting the modified nucleic acid with the labelled antibodies; and means for measuring for the presence and/or extent of the presence of the label as an indication of the presence or extent of presence of the suspect nucleotide.

The probe itself forms one aspect of the invention because of the facility which it provides in an assay such as the assay of the present invention. Such a probe may be a single or partially denatured double stranded DNA or RNA molecular genetic probe comprising at least one modified base. The base is modified while it is attached to the DNA r RNA in a one-step addition, and without the use of mercury or a linker arm. The probe is further adpated to hybridize with a complementary DNA or RNA strand. Hybridization per se is a known technique and is generally described in numerous publications, see, for example, European Patent Application 82102673.9, published October 13, 1982, page 7. Of course, the probe of the invention acts as an antigen to produce antibodies which find particular use as ;abelling agents according to the invention. The probe itself may be modified while it is single or double stranded, or when it is partially denatured when a modification technique such as that disclosed herein is used.

The instant invention finds particular application in assaying for the presence and/or quantity of nucleic acid from organisms which contain, either single or double stranded nucleic acid, such as DNA and RNA, or their nucleotides. The nucleic acids being assayed according to the invention have at least 10 bases, and preferably at least 50 bases. The present invention does not require that the nucleic acid being assayed for comprises part of the coding sequence for a structural gene. However, although the method extends to techniques for the assay of DNA sequences which do not code for a peptide, (such as tRNA and rRNA coding sequences, other non-translated RNA gene products, and controlling sequences in the DNA such as promoters etc.) in a preferred embodiment of the invention the sequence which is being assayed for is a whole or part coding sequence for a structural gene directing the synthesis of an enzyme.

It should be noted that when assaying for nucleic acids in organisms containing single stranded nucleic acids (such as in the ssRNA viruses), hybridization can be performed directly without denaturation.

Prior to assaying cell-contained DNA, the cells must be lysed. To accomplish this, a stranded lysing technique is used; or, if desired, a lysing technique of the type described in U.S.P.N. 4,358,535 to FALKOW et al/. may be used.

An alternative lysing technique according to the invention includes identifying by number colonies on agar, transferring them into separate test tubes containing lysing and denaturing solution, and transferring the lysed and denatured contents onto either nitrocellulose or activated paper.

When assaying for nucleic acid from or ganisms containing double stranded nucleic acids, a preliminary splitting (denaturation) of the nucleic acid is required so as to render it at least partially single stranded. Such techniques are known and do not form a part of the present invention. Examples of such techniques are disclosed, for instance, in Gergen, J. P., Stern, R.H. and Wensink, P.C. (1980) Nucleic Acids Res., 7,2115-2136.

The suspect nucleic acid material may be assayed while suspended in a liquid or when fixed to a suspender or solid substrate, e.g., filter paper or cellulose. The first step fo the assay involves hybridizing the suspect nucleic acid with a test probe which is as specific as possible to the substance being assayed. Thus, it is preferred that the probe of the invention be a nucleic acid having at least 10 and preferbly at least 50 bases.

The probe itself comprises a single strand, or partially denatured double strand, of nucleic acid, either DNA or RNA, which is either extracted from plant or animal tissue or cloned, both techniques being well known and disclosed, for instance, in Krist et al. (1981), Proc. Natl. Acad. Sci., US, 78, 2772-2776.

The proble itself is modified, preferably prior to hybridization, by a one-step technique which does not require nicking or enzymatic action on the nucleic acid strand. Instead the probe is modified directly while it is either single or double stranded, or the denatured portion of a partially denatured double strand is modified.

A variety of different modifications may be performed to provide the modified probe. The object of the modification technique is to provide a probe which is an effective antigen, and any modification achieving this aim will be satisfactory provided that it does not interfere with, or substantially hinder the hybridization step in which the probe will be used. Thus, according to the invention the modifying compound may be a compound selected from the group capable of modifying the nucleic acid such that it is substituted with an alkyl group, including alkyl groups, having three or less, or three or more carbon atoms, a sulfono group,; a nitroso group, or nitrophenol group. Many other modifying agents may also be used. Specifically, N-methyl nitrosourea, N-ethyl nitrosourea, N-propyl nitrosourea, and N-butyl nitrosourea may be considered for use as alkylating agents, although N- ethyl nitrosourea is preferred. An alkylation technique of this type is known and is diclosed in M.F.

Rajewsky et al., Immunological Detection, etc., Carcinogen Fundamental Mechanism and Environmental Effects, 207-218, 1980, D. Reidel Publishing Co., although it should be remembered that such techniques have not previously been used as part of a hybridization assay. In alkylating with these compounds substitution occurs at various positions on the nucleic acid.

Compounds providing sulfone groups may include bisulphite, or a combination of the same and, for example, 0-methylhydroxylamine as in Poverenny et al., supra, which discloses one possible sulfonation technique. Sulfonation occurs as a 6 sulpho 5, 6 dihydro 4-me-thoxyaminopyrimidon-2 position on the nucleic aicd.

Compounds providing nitroso and nitrophenyl groups, as well as any other compounds which modify by addition reaction may also be used.

The range of modifications which can be carried out allows a variabillity in the chemical species which are employed in the assay technique. It is therefore possible to select an assay which does not substantially interfere with or otherwise substantially peturb the system being investigated.

Once modified by the addition of a compound of the types listed above, the probes are ready for hybridization, and are then contacted with the suspect sample under conditions allowing hybridization to occur.

This contact can be performed sing a variety of techniques, such as liquid-liquid contact, as well as contact on a substrate support, etc. Such contact techniques are exemplified by P.S. Thomas, Proc, Natl. Acad. Scie., 1980, 5201-5205.

According to a preferred technique, the suspect DNA or RNA is affixed to a cellulosic support, such as on a piece of filter paper. Specifically, a cellulosic or nitrocellulosic filter support paper may be used which may or may not be porous. One such material is available from Neww England Nuclear as Gene Screen hybridization transfer membrane. Other support material such as nonporous plastics may also be used. The probe is then contacted to the support so as to permit it to hybridize with any suspect DNA and/or RNA suspended on the support. This may be accomplished through capillary action by simply applying filter paper to a gel having the probes distributed therein.

After hybridization, and preferably while the hybridized complex is still on the filter paper, the hydribized complex is tagged with a labelling agent which makes an assay possible.

The labelling agent used may be an enzyme, an enzyme-antibody complex, a radioactively tagged antibody or enzyme etc. To avoid the use of radioactive materials, it is preferred that an EIA (Enzyme Immunoassay) or like system be used in conjunction, for example, with a colorimetric analysis technique. As in standard EIA techniques, amplification, and the like may be used. One advantage of the invention is that the modified probes are particularly good antigens, and it is preferred for purposes of the invention that the labelling agent used be an antibody, complexed with an enzyme. The antibodies are obtained by standard techniques such as are described by Poverenny et al., supra, or Muller R. Adamkiewicz and M.F. Rajewsky, 10 IARC Scientific Publ, 39 pp. 436-479. The enzymes used may be peroxidase, B-galactosidase, hyaluronidase, and alkaline phosphatase as well as others disclosed on pages 52-59 and 61-64 of European Application S.N. 80303405.7, published July 21, 1981; and may be complexed with the antibodies by means of standard techniques.

The invention will now be described by way of example.

EXAMPLE I

Detection of SV40 Sequences In SV40 Infected Cells

A) Preparation of Cultures

CV-I Cells were infected with SV40 (Simian Virus 40) and cultivated further for 16 hours. The cells were then trypsinized and diluted in a phosphate buffered saline solution to obtain a final count on the order of 1,000 cells. This suspension was then filtered on nitrocellulose filters and the DNA denatured as described in S. Lavi & S. Etkin, Carcinogenesis 2 (1981) 417-423 and in Gall J.G. & Pardue M.L. (1969) P. NAS 63 378-383. After preincubation at 67°C for 4 hours in Denhardt Buffer (Denhardt D.T. "A membrane filter technique for the detection of complementary DNA) (1966) Biochem, Biophys. Res. Com. 23 641-646), concentrated three times, the filters were contacted with the same buffer containing denatured calf thymus DNA (SIGMA) carrier at 100 micrograms/ml concentration as well as a 5 micrograms/ml7 quantity of SV40 DNA modified by alkylation with ethyl nitrosourea as indicated below.

B) Alkylation of SV 40 DNA

Pure SV40 DNA ethylated using N-ethyl nitrosourea following the procedure described in R. Muller & Rajewsky M.F. (1978) Naturforsch 33 pp., 897-901, and the DNA precipitated by ethanol precipitation, was washed twice by reprecipitation and freeze dried. This material constitutes the molecular probe.

C) Antibody Formation

Antibodies to ethylated DNA were produced by using calf thymus DNA processed as above for ethylation. Antibody formation was done by repeated injection of ethylated calf thymus DNA (250-1000 micrograms/ml) with the addition of aluminium hydroxide and complete Freund adjuvent to rabbits subcutaneously and in the hind foot. This was repeated and followed by intravenous injection of booster until the titer of antibody obtained was judged sufficient. Alternatively monoclonal antibodies were used.

D) Isolation of Specific Antibodies

DNA affinity procedure was used to which antibodies directed against non-modifed DNA were bound after 2 hours of incubation. Proteins which were not bound were passed through an affinity column containing ethylated bases and were then further eluted.

E) Demonstration of the SV 40 Sequences

Filter prepared as in a) were exposed to probes prepared as in b) for hybridization procedure with the modified probe as described in Lavi et al (1981) supra. After completion of the hybridization procedure, filters were incubated with a protein solution (10% normal, inactivated serum); washed in saline; and covered with a mixture of anti-ethylated DNA antibodies as in c) for 2 hours at 37°C. The filters were then washed and exposed to commercially available peroxidase conjugated goat-anti rabbit immunoglobulin (Miles-Yeda) and a color reaction developed by standard procedures. The appearance of a staining reaction showed the presence of SV 40 DNA, and its extent showed the amount of such sequences.

It is envisaged that the present invention can be embodied in alternative protocols to that given by way of example above. The following are illustrative embodiments of the alternative protocols which have been envisaged.

PROTOCOL II-Use of modified molecular probes for the identification of specific genes after gel electrophoresis and blotting

DNA from E. Coil bacteria which have been transformed by a plasmid containing a cloned gene (e.g. globin) is cut by restriction enzymes and processed for electrophoresis and transfer as in Southern E.M. (1975) J. Mol Biol 98, 503-517.

The same plasma will be processed separately for alkylation or sulfonation as indicated in Example Ib and will constitute the modified molecular probe.

Hybridization between the modified molecular probe and the blotted DNA fragments can be performed as in Southern E.M. (1975) supra (in which the hybridization material was radioactive).

After completion of hybridization, antibodies as in example Ic will be used and processed to obtain a color reaction as in example Ic. Only the bands containing part of the globin gene bind with the modified molecular probe and as a consequence with antibodies against it to give rise to a stained reaction. This same procedure can be used for cellular genes and is not restriuced to plasmids. Likewise, the procedure is not restricted to prokaryotic cells and can be applied to nucleated (eukaryotic) cells.

PROTOCOL III-Identification of bacterial colonies

Bacterial colonies on an Agar plate are transferred onto nitrocellulose filters or activated paper by blotting and a few "prints" obtained and processed as described by Gall J.G. & Pardue M.L.-(1969) Proc. Nat. Adad, Sci. U.S. Washington 63 378383, for denaturation. Modified molecular probes consisting of DNA fragments specific for one given bacterial strain are made as in Example Ib. Each of the modified molecular probes are applied for hybridization on a separate "print" of the bacterial colonies and processed for antibody reaction as in Example I.

A stained reaction will appear and appear only at the spot corresponding to the bacterial colony possessing a gene complementary to the known modified molecular probe permitting the direct identification of the nature of the bacterial colony.

PROTOCOL IV-Identification of the presence of a given DNA in a mixture

Friend Leukemia Cells which can be induced to differentiate and produce hemoglobin by addition of DMSO are used in this experiment.

Whole cytoplasmic RNS is isolated at different times after addition of the differentiation factor and fixed on an activated paper as in Alwine J.C., Kemp D. and Stark G (1977) Proc. Nat. Acad. Sci. U.S. 74, 5350-5354. A plasmid containing all or part of the globin gene and modified as in Example Ib is then used by hybridization against these spots and further processed for antibody reaction and stained reaction as in Example I. The intensity of coloration will be a direct indication of the quantity of globin messenger RNA present in the cytoplasm of these cells at the different stages of differentiation.

PROTOCOL V-Use of double modification to distinguish between two genes

As in Example I, cells suspected of containing either Herpes Virus Type I, Herpes Virus Type II or of being free of these viral genes are processed onto nitrocellulose filters. These cells can be from a patient (clinical sample) or from cultured cells (research sample). Two modified molecular probes are used.:

Herpes I: A plasmid containing a fragment of Herpes I DNA which will not hybridize with Herpes II DNA is obtained and modified by ethyl nitrosourea as in Example I. Antibodies to the modification are raised in rabbits.

Herpes II: A plasmid containing a frag ment of Herpes II DNA which will not hybridize with Herpes I DNA is obtained and modified by sulfonation as in Poverenny et al. (1979) Molecular Immunology 16 313-316.

Antibodies against sulfonated nucleic acid are raised in goats.

A mixture of two modified molecular probes is then contacted with the suspected sample for hybridization as described in Example Ia.

Two "second" antibodies are now used:

a) Antirabbit immunoglobulin coupled to peroxidase which produces a green dye when the correct substrate is present (ABTS) (22'azino-di-3-ethyl (benthiazolin sulfonic acid)

b) Antigoat immumoglobulin coupled to beta galactosidase which gives a blue dye when the correct substrate is present (X gal).

A mixture of the two antibodies is used to determine whether thw anti-goat or anti-rabbbit is attached to the hybridized molecules. The possibilities are differentiated by the colour of the final product.

The presence of the green colour after complete reaction indicates the presence of the Herpes I genes in the suspected cells.

The presence of a blue colour after complete reaction indicates the presence of the Herpes II genes in the suspected cells. The presence of the two colours shows the presence of the two genes. The absence of a stained reaction showes that the tissue is free of Herpes I and II genes.

The same kind of determination can be made with more than two modified molecular probes and used to test both body fluids and tissues.

## Claims

1. A method of assaying for the presence or quantity of a suspect nucleic acid in a suspect sample containing a single stranded nucleic acid sequence, comprising the steps of:

(a) contacting said suspect sample with a modified nucleic acid probe containing a single stranded nucleic acid sequence, complementary to a nucleic acid sequence of the suspect nucleic acid, to form a hybridized complex, said modified nucleic acid probe having been formed by a one-step modification process selected from the group consisting of alkylation, sulfonation, nitrosonation, or nitrophenylation;

(b) contacting said hybrydized complex with antibodies specific to the modified portion of said modified nucleic acid probe to form an antibody-bound complex; and

(c) measuring for the presence and/or extent of the presence of said antibodies as an indication of the presence or extent of said suspect nucleic acid.

2. The method defined by Claim 1 comprising further contacting in step (b) said antibody-bound complex with a second non-radioactively labelled antibody specific to said antibody-bound complex.

3. The method as defined by Claim 1 wherein said antibody is monoclonal antibody.

4. The method as defined by Claim 1 wherein said modified nucleic acid probe has at least 10 bases.

5. The method as defined by Claim 1 wherein said suspect nucleic acid comprises a sequence having at least 10 bases.

6. The method as defined by claim 1 wherein said suspect nucleic acid codes for an enzyme.

7. The method as defined by claim 1 wherein said suspect nucleic acid is a viral DNA or RNA.

8. The method as defined by claim 7 wherein said suspect nucleic acid is viral Herpes DNA.

9. The method as defined by claim 1 wherein said modified nucleic acid has been modified by alkylation using a compound selected from the group consisting of N-methyl nitroso urea, N-ethyl nitroso urea, N-propyl nitroso urea, N-butyl nitroso urea.

10. The method as defined by claim 9 wherein said modified nucleic acid has been modified by alkylation of the 0-6 guanidine.

11. The method as defined by claim 9 wherein said modified nucleic acid is butylated.

12. The method as defined by claim 9 wherein said modified nucleic acid is ethylated.

13. The method as defined by claim 1 wherein said modified nucleic acid has been modified by sulfonation using a compound selected from the group consisting of bisulfites, 0-methyl hydroxylamine and combinations thereof.

14. The method as defined by claim 1 comprising performing step b) wherein said antibodies are labelled with an enzyme selected to provide a colour reaction.

15. The method as defined in claim 14 wherein said enzyme is selected from the group consisting of peroxidase, betagalactosidase, hyalyronidase, and alkaline phosphatase.

16. The method as defined by claim 1 wherein said antibodies are labelled with an agent adapted to signal the presence of said antibodies.

17. The method as defined by claim 1 wherein said suspect nucleic acid is single stranded RNA.

18. The method as defined by claim 1 wherein said suspect nucleic acid is normally double stranded and was at least partially denatured prior to contact with said modified nucleic acid probe according to step a).

19. The method as defined by claim 1 wherein said modified nucleic acid probe is double stranded and is at least partially denatured prior to contact with said suspect nucleic acid probe according to step a).

20. A kit for testing for the presence of nucleic acid in a suspect sample comprising:
   a) a supply of modified nucleic acid probes hybridizable with said suspect nucleic acid, said modified nucleic acid probes having been formed by a one-step modification process selected from the group consisting of alkylation, sulfonation, nitrosonation and nitrophenylation;
   b) means for contacting said suspect sample with said modified nucleic acid probe to form said hybridized complex;
   c) a supply of antibodies selected to complex with at least the modified protion of said modified nucleic acid probe;
   d) means for contacting said modified nucleic acid with said labelled antibodies;
   e) a supply of labelling agents adapted to signal the presence of said antibody; and
   f) means for measuring for the presence and/or extent of the presence of said label as an indication of the presence or extent of presence of said suspect nucleic acid.

21. A method of assaying for the presence or quantity of a suspect nucleic acid in a suspect sample containing a single stranded nucleic acid sequence, comprising the steps of:
   a) contacting, under hybridizing conditions, said suspect sample with a plurality of modified nucleic acids, said modified nucleic acids having been formed by a one-step modification process selected from the group consisting of alkylation, sulfonation, nitrosonation, or nitrophenylation and each of said modified nucleic acids being hybridizable with said suspect nucleic acid depending upon identity of said suspect nucleic acid to from a hybridized complex;
   b) contacting said hybridized complex with antibodies specific to at least the modified portion of said modified nucleic acid to form an antibody bound complex; and
   c) measuring for the presence and/or extent of the presence of said antibodies as an indication of the presence or extent of presence of said suspect nucleic acid.

**Revendications**

1. Méthode pour tester la présence ou la quantité d'un acide nucléique suspect dans un échantillon suspect contenant une séquence d'acide nucléique monocaténaire, comprenant les stades de:
   (a) mise en contact de l'échantillon suspect avec une sonde d'acide nucléique modifié contenant une séquence d'acide nucléique monocaténaire complémentaire d'une séquence d'acide nucléique de l'acide nucléique suspect, pour former un complexe hybridé, ladite sonde d'acide nucléique modifié ayant été formée par un procédé de modification en une étape choisi dans le groupe constitué par l'alkylation, la sulfonation, la nitrosonation ou la nitrophénylation
   (b) mise en contact dudit complexe hybridé avec des anticorps spécifiques pour la partie modifiée de ladite sonde d'acide nucléique modifié pour former un complexe lié à l'anticorps et
   (c) la mesure de la présence et/ou de l'étendue de la présence desdits anticorps comme une indication de la présence ou de la quantité dudit acide nucléique suspect.

2. Méthode définie par le Revendication 1 comprenant en outre la mise en contact dans l'étape (b) dudit complexe lié à l'anticorps avec un second anticorps marqué de manière non-radioactive spécifique pour ledit complexe lié à l'anticorps.

3. Méthode telle que définie par la Revendication 1, dans laquelle ledit anticorps est un anticorps monoclonal.

4. Méthode telle que définie par la Revendication 1, dans laquelle ladite sonde d'acide nucléique modifié a au moins 10 bases.

5. Méthode telle que définie par la Revendication 1, dans laquelle ledit acide nucléique suspect comprend une séquence ayant au moins 10 bases.

6. Méthode telle que définie par la Revendication 1, dans laquelle ledit acide nucléique suspect code pour une enzyme.

7. Méthode telle que définie par la Revendication 1, dans laquelle ledit acide nucléique suspect est de l'ADN ou de l'ARN viral.

8. Méthode telle que définie par la Revendication 7, dans laquelle ledit acide nucléique suspect est l'ADN viral de l'Herpes.

9. Méthode telle que définie par la Revendication 1, dans laquelle ledit acide nucléique modifié a été modifié par une alkylation en utilisant un composé choisi dans le groupe constitué par la N-méthyl nitroso urée, la N-éthyl nitroso urée, la N-propyl nitroso urée et la N-butyl nitroso urée.

10. Méthode telle que définie dans la Revendication 9, dans laquelle ledit acide nucléique modifié a été modifié par une alkylation de la 0-6 guanidine.

11. Méthode telle que définie dans la Revendication 9, dans laquelle ledit acide nucléique modifié est butylé.

12. Méthode telle que définie dans la Revendication 9, dans laquelle ledit acide nucléique modifié est éthylé.

13. Méthode telle que définie par la Revendication 1, dans laquelle ledit acide nucléique modifié a été modifié par une sulfonation en utilisant un composé choisi dans le groupe constitué par les bisulfites, l'0-méthyl hydroxylamine et leurs combinaisons.

14. Méthode telle que définie par la Revendication 1, comprenant la réalisation de l'étape (b) dans laquelle des anticorps sont marqués avec une enzyme choisie de manière à fournir une réaction de coloration.

15. Méthode telle que définie par la Revendication 14, dans laquelle ladite enzyme est choisie dans le groupe constitué par la peroxydase, la bêtagalactosidase, l'hyalyronidase et l'alcaline phosphatase.

16. Méthode telle que définie par la Revendication 1, dans laquelle lesdits anticorps sont marqués avec un agent adapté pour signaler la présence desdits anticorps.

17. Méthode telle que définie par la Revendication 1, dans laquelle ledit acide nucléique suspect est de l'ARN monocaténaire.

18. Méthode telle que définie par la Revendication 1, dans laquelle ledit acide nucléique suspect est normalement bicaténaire et a été au moins partiellement dénaturé avant d'être mis en contact avec ladite sonde d'acide nucléique modifié suivant l'étape (a).

19. Méthode telle que définie par la Revendication 1, dans laquelle ladite sonde d'acide nucléique modifié est bicaténaire et est au moins partiellement dénaturée avant d'être mise en contact avec ladite sonde d'acide nucléique suspect suivant le stade (a).

20. Trousse pour tester la présence d'acide nucléique dans un échantillon suspect comprenant:

(a) un approvisionnement en sondes d'acides nucléiques modifiés pouvant s'hybrider avec ledit acide nucléique suspect, lesdites sondes d'acides nucléiques modifiés ayant été formées par un procédé de modification en une étape choisie dans le groupe constitué par l'alkylation, la sulfonation, la nitrosonation et la nitrophénylation.

(b) un moyen pour mettre en contact ledit échantillon suspect avec ladite sonde d'acide nucléique modifié pour former ledit complexe hybridé

(c) un approvisionnement en anticorps choisis pour se complexer avec au moins la partie modifiée de ladite sonde d'acide nucléique modifié

(d) un moyen pour mettre en contact ledit acide nucléique modifié avec lesdits anticorps marqués

(e) un approvisionnement en agents de marquage adaptés pour signaler la présence dudit anticorps et

(f) un moyen pour mesurer la présence et/ou l'étendue de la présence de ladite marque comme une indication de la présence ou de l'étendue de la présence dudit acide nucléique suspect.

21. Méthode pour tester la présence ou la quantité d'un acide nucléique suspect dans un échantillon suspect contenant une séquence d'acide nucléique monocaténaire, comprenant les stades de:

(a) mise en contact, dans des conditions d'hybridation, dudit échantillon suspect avec une pluralité d'acides nucléiques modifiés, lesdits acides nucléiques modifiés ayant été formés par un procédé de modification en une étape choisi parmi le groupe constitué par l'alkylation, la sulfonation, la nitrosation ou la nitrophénylation et chacun desdits acides nucléiques modifiés pouvant s'hybrider avec ledit acide nucléique suspect en fonction de l'identité dudit acide nucléique suspect pour former un complexe hybridé;

(b) la mise en contact dudit complexe hybridé avec des anticorps spécifiques pour au moins la partie modifiée dudit acide nucléique modifié pour former un complexe lié à l'anticorps et

(c) la mesure de la présence et/ou de l'étendue de la présence desdits anticorps comme une indication de la présence ou de l'étendue de la présence dudit acide nucléique suspect.

**Patentansprüche**

1. Verfahren zur qualitativen oder quantitativen Bestimmung einer vermuteten Nucleinsäure in einer verdächtigen Probe, die eine einsträngige Nucleinsäuresequenz enthält, wobei das Verfahren folgende Stufen umfaßt:

(a) Die verdächtige Probe wird mit einer modifizierten Nucleinsäuresonde versetzt, die eine einsträngige Nucleinsäuresequenz enthält, welche komplentär zur Nucleinsäuresequenz der vermuteten Nucleinsäure ist, wobei ein hybridisierter Komplex gebildet wird und wobei die modifizierte Nucleinsäuresonde durch ein einstufiges Modifizierungsverfahren gebildet worden ist, das aus der aus Alkylierung, Sulfonierung, Nitrosierung oder Nitrophenylierung bestehenden Gruppe ausgewählt ist;

(b) der hybridisierte Komplex wird mit für den modifizierten Teil der modifizierten Nucleinsäuresonde spezifischen Antikörpern unter Bildung eines antikörpergebundenen Komplexes versetzt; und

(c) die Anwesenheit und/oder die anwesende Menge der Antikörper wird als Anzeige für die Anwesenheit oder die anwesende Menge der vermuteten Nucleinsäure gemessen.

2. Verfahren nach Anspruch 1, dadurch gekennzeichnet, daß zusätzlich in Stufe (b) der antikörpergebundende Komplex mit einem zweiten, nicht-radioaktiv markieren, für den antikörpergebundenen Komplex spezifischen Antikörper versetzt wird.

3. Verfahren nach Anspruch 1, dadurch gekennzeichnet, daß der Antikörper ein monoklonaler Antikörper ist.

4. Verfahren nach Anspruch 1, dadurch gekennzeichnet, daß die modifizierte Nucleinsäuresonde mindestens 10 Basen aufweist.

5. Verfahren nach Anspruch 1, dadurch gekennzeichnet, daß die vermutete Nucleinsäure eine Sequenz mit mindestens 10 Basen umfaßt.

6. Verfahren nach Anspruch 1, dadurch gekennzeichnet, daß die vermutete Nucleinsäure für ein Enzym kodiert.

7. Verfahren nach Anspruch 1, dadurch gekennzeichnet, daß die vermutete Nucleinsäure eine virale DNA oder RNA ist.

8. Verfahren nach Anspruch 7, dadurch gekennzeichnet, daß die vermutete Nucleinsäure eine virale Herpes-DNA ist.

9. Verfahren nach Anspruch 1, dadurch gekennzeichnet, daß die modifizierte Nucleinsäure durch Alkylierung modifiziert worden ist, und zwar unter Verwendung einer aus der aus N-Methylnitrosoharnstoff, N-Ethylnitrosoharnstoff, N-Propylnitrosoharnstoff und N-Butylnitrosoharnstoff bestehenden Gruppe ausgewählten Verbindung.

10. Verfahren nach Anspruch 9, dadurch gekennzeichnet, daß die modifizierte Nucleinsäure durch Alkylierung von Guanidin in 0-6-Stellung modifiziert worden ist.

11. Verfahren nach Anspruch 9, dadurch gekennzeichnet, daß die modifizierte Nucleinsäure butyliert ist.

12. Verfahren nach Anspruch 9, dadurch gekennzeichnet, daß die modifizierte Nucleinsäure ethyliert ist.

**13.** Verfahren nach Anspruch 1, dadurch gekennzeichnet, daß die modifizierte Nucleinsäure durch Sulfonierung modifiziert worden ist, und zwar unter Verwendung einer aus der aus Bisulfiten, O-Methylhydroxylamin und deren Gemischen bestehenden Gruppe ausgewählten Verbindung.

**14.** Verfahren nach Anspruch 1, dadurch gekennzeichnet, daß in Stufe (b) die Antikörper mit einem speziell zur Erzeugung einer Farbreaktion ausgewählten Enzym markiert sind.

**15.** Verfahren nach Anspruch 14, dadurch gekennzeichnet, daß das Enzym aus der aus Peroxidase, Beta-Galaktosidase, Hyaluronidase und alkalischer Phosphatase bestehenden Gruppe ausgewählt wird.

**16.** Verfahren nach Anspruch 1, dadurch gekennzeichnet, daß die Antikörper mit einem speziellen Mittel zum Anzeigen der Anwesenheit der Antikörper markiert werden.

**17.** Verfahren nach Anspruch 1, dadurch gekennzeichnet, daß die vermutete Nucleinsäure eine einsträngige RNA ist.

**18.** Verfahren nach Anspruch 1, dadurch gekennzeichnet, daß die vermutete Nucleinsäure normalerweise doppelsträngig ist und mindestens teilweise vor dem Kontakt mit der modifizierten Nucleinsäuresonde gemäß Stufe (a) denaturiert worden ist.

**19.** Verfahren nach Anspruch 1, dadurch gekennzeichnet, daß die modifizierte Nucleinsäuresonde doppelsträngig ist und mindestens teilweise vor dem Kontakt mit der vermuteten Nucleinsäure gemäß Stufe (a) denaturiert worden ist.

**20.** Testsatz zur Bestimmung der Anwesenheit von Nucleinsäure in einer verdächtigen Probe, umfassend:
a) Einen Vorrat an modifizierten Nucleinsäuresonden, die mit der vermuteten Nucleinsäure hybridisierbar sind, wobei die modifizierten Nucleinsäuresonden durch ein einstufiges Modifizierungsverfahren gebildet wurden, das aus der aus Alkylierung, Sulfonierung, Nitrosierung und Nitrophenylierung bestehenden Gruppe ausgewählt ist;
b) Mittel zum Zusammenbringen der verdächtigen Probe mit der modifizierten Nucleinsäuresonde zwecks Bildung des hybridisierten Komplexes;
c) einen Vorrat an für die Komplexbildung mit mindestens dem modifizierten Teil der modifizierten Nucleinsäuresonde ausgewählten Antikörpern;
d) Mittel zum Zusammenbringen der modifizierten Nucleinsäuresonde mit den markierten Antikörpern;
e) einen Vorrat an speziellen Markierungsmitteln für die Anzeige der Anwesenheit des Antikörpers; und
f) Mittel zur Messung der Anwesenheit und/oder der anwesenden Menge des Markierungsmittels als einer Anzeige für die Anwesenheit oder die anwesende Menge der vermuteten Nucleinsäure.

**21.** Verfahren zur qualitativen oder quantitativen Bestimmung einer vermuteten Nucleinsäure in einer verdächtigen Probe, die eine einsträngige Nucleinsäuresequenz enthält, wobei das Verfahren folgende Stufen umfaßt:
a) Die verdächtige Probe wird unter Hybridisierungsbedingungen mit einer Vielzahl modifizierter Nucleinsäuren versetzt, wobei die modifizierten Nucleinsäuren durch ein einstufiges Modifizierungsverfahren, ausgewählt aus der aus Alkylierung, Sulfonierung, Nitrosierung oder Nitrophenylierung bestehenden Gruppe, gebildet worden sind und jede der modifizierten Nucleinsäuren mit der vermuteten Nucleinsäure, abhängig von der Identität der vermuteten Nucleinsäure, unter Bildung eines hybridisierten Komplexes hybridisierbar ist;
b) der hybridisiere Komplex wird mit für mindestens den modifizierten Teil der modifizierten Nucleinsäure spezifischen Antikörpern unter Bildung eines antikörpergebundenen Komplexes versetzt; und
c) die Anwesenheit und/oder die anwesende Menge der Antikörper wird als Anzeige für die Anwesenheit oder die anwesende Menge der vermuteten Nucleinsäure gemessen.